# EUROPEAN PATENT SPECIFICATION

(11) **EP 1 231 220 B1**
(45) Date of publication and mention of the grant of the patent: **13.05.2009**
(21) Application number: 02001469.2
(22) Date of filing: 22.01.2002
(51) Int. Cl.: C07K 14/755, C12N 9/64, C12N 15/67, C12N 15/85

(54) **Modified factor VIII cDNa and its use for the production of factor VIII**
Modifizierte Faktor VIII cDNA und ihre Verwendung zur Produktion von Faktor VIII
ADNc modifié du facteur VIII et son utilisation pour la préparation du facteur VIII

(30) Priority: 09.02.2001 EP 01102810
(43) Date of publication of application: 14.08.2002
(73) Proprietor: Négrier, Claude, 69437 Lyon Cédex 03 (FR)
(72) Inventor: Négrier, Claude, 69540 Irigny (FR); Plantier, Jean-Luc, 69520 Gringy (FR); Rodrigeuz, Marie Hèléne, 38300 Nivolas Vermelle (FR)
(74) Representative: Hirsch & Associés

(56) References cited:
- EP-A- 1 038 959
- EP-A- 1 048 726
- PLANTIER J-L ET AL: "A combination of truncated factor IX intron I highly improves FVIII production" BLOOD, W.B. SAUNDERS, PHILADELPHIA, VA, US, vol. 94, no. 10 SUPPL 1 PART 1, 15 November 1999 (1999-11-15), page 454a XP002148565 ISSN: 0006-4971
- UZAN G ET AL: "TISSUE-SPECIFIC EXPRESSION OF THE PLATELET GPIIB GENE" JOURNAL OF BIOLOGICAL CHEMISTRY, AMERICAN SOCIETY OF BIOLOGICAL CHEMISTS, BALTIMORE, MD, US, vol. 266, no. 14, 15 May 1991 (1991-05-15), pages 8932-8939, XP000986104 ISSN: 0021-9258
- BAATOUT SARAH ET AL: "Protein content and number of nucleolar organizer regions are enhanced during phorbol ester-induced differentiation of cultured human megakaryocytic cells." ANTICANCER RESEARCH, vol. 19, no. 4B, July 1999 (1999-07), pages 3229-3236, XP001068515 ISSN: 0250-7005
- PLANTIER JEAN-LUC ET AL: "A factor VIII minigene comprising the truncated intron I of factor IX highly improves the in vitro production of factor VIII." THROMBOSIS AND HAEMOSTASIS, vol. 86, no. 2, August 2001 (2001-08), pages 596-603, XP001068506 ISSN: 0340-6245

## Description

### FIELD OF THE INVENTION

This invention is directed to a modified Factor VIII cDNA and its use for the improvement of the Factor VIII production.

### BACKGROUND OF THE INVENTION

Factor VIII (FVIII) is a X-linked gene product implicated in the blood coagulation cascade. The factor VIII is synthetized as a 2351 amino acid single-chain polypeptide having the domain structure A1-A2-B-A3-C1-C2 and comprising a 19 amino acid signal peptide (Gitschier J. et al., 1984; Toole, J.J, et al., 1984; Vehar, G.A. et al., 1984). Plasma FVIII is a heterodimer consisting of a carboxy-terminal derived light chain of 80 kDa in a metal-ion dependent association with a variable sized amino-terminal heavy chain (200-90 kDa; Andersson et al., 1986). Absence or defi ciency of FVIII causes severe bleeding disorders called hemophilia A.

The level of FVIII production remains low after cell transfection compared to other genes. Three reasons have been identified so far: (1) FVIII mRNA is inefficiently produced, (2) FVIII translocation from endoplasmic reticulum to Golgi apparatus is low and (3) FVIII is sensitive to proteolysis, Therefore, the improvement of FVIII transgenes is an important challenge for hemophilia A gene therapy.

It has already been proposed in the European patent application 99 104 050.2 to modify the FVIII cDNA by deleting the B-domain of the wild type cDNA and by inserting a truncated FIX intron 1 in different locations of the Factor VIII cDNA. Such modified Factor VIII cDNA may be used for a higher yield production of FVIII in vitro as well as in a transformation vector for gene therapy. A cDNA bearing the FIX truncated intron 1 in both the intron 1 and intron 13 locations led to the highest FVIII production after transfection of CHO and HepG2 cell lines. This recombinant FVIII was a biologically active protein.

In order to further improve the yield in the gentechnological production of Factor VIII the present invention is directed to the expression of Factor VIII in hematopoietic cells and especially in platelets using the tissue-specific promoter of the glycoprotein II b (GPIIb).

The feasitility of this approach was demonstrated, first by use of the hematopoietic cell line HEL. The human erythroleukemia cell line is known to express an erythroid phenotype (Martin et al., 1982) but also some megakaryocytic markers such as platelet membrane glycoproteins (Tabilio et al., 1984). Upon induction by phorbol-12-myristate-13-acetate (PMA), HEL cell line expresses increased amounts of megakaryocytic proteins like glycoproteins IIb/IIIa, Platelet factor 4, or von Willebrand factor (vWF) (Long and coll., 1990).

Another hematopoietic cell line, Dami, established from the blood of a patient with a megakaryoblastic leukaemia, appears to be a pure population of megakaryocyte-like cells (Greenberg et al., 1988). Cultured Dami cells express platelet glycoproteins GPIb and GPIIb/IIIa. After PMA stimulation, surface expression of these two platelet glycoproteins, and vWF synthesis were increased. These changes were associated with a decrease in the proliferation of the stimulated Dami cells (Greenberg et al., 1988; Ballen et al., 1996; von der Vuurst et al., 1998). The multimerin molecule, which colocalizes with vWF in platelet α-granules, was shown to be synthetized in PMA-stimulated Dami cells where it presented a granular distribution (Hayward et al., 1993). The same results were obtained with the plasminogen activator inhibitor type I and vWF (Hill et al., 1996). Dami cells were used to study the megakaryocyte-specific expression of FVIII under the GPIIb promoter control.

### SUMMARY OF THE INVENTION

The present invention disposes the ability of hematopoietic cell lines to produce an active FVIII molecule. It could be demonstrated that Dami cells transfected with the GPIIb constructs are able to synthetize FVIII and that FIX intron 1 sequences increase dramatically the production of Factor VIII.

Thus, the present invention is a Factor VIII cDNA wherein the B-domain of the wild-type factor VIII cDNA has been deleted and wherein a truncated factor IX intron 1 has been inserted into the factor VIII introns 1 and 13, characterized in that it contains, as a promoter, the tissue-specific promoter of the glycoprotein IIb (GPIIb).

The present invention also relates to a process for the production of Factor VIII, characterized in that the production is performed in the cell line HEL or in the cell line Dami using the above described Factor VIII cDNA.

### DESCRIPTION

A modified Factor VIII cDNA has been found wherein the B-domain of the wild type factor cDNA has been deleted and a truncated Factor IX intron has been inserted in two locations of the Factor VIII cDNA containing as a promoter a cDNA which is suitable for the expression in-hematopoietic cell lines and specifically in platelets. The promoter of the human platelet glycoprotein IIb (GPIIb) is preferred as a promoter. The modified Factor VIII cDNA of the present invention contains the truncated Factor IX intron 1 in the Factor VIII introns 1 and 13.

A further object of the invention is a process for the production of Factor VIII in the cell lines HEL or Dami using the above-mentioned modified Factor VIII cDNA. Preferred is a process wherein the production of Factor VIII is stimulated by an inducer. The best results have been obtained when phorbol-12-myristate-13-acetate (PMA) was used.

### Materials and Methods

*Vectors:* The pcDNA3-FVIII and pcDNA3-FVIII I1+13 were the same vectors as disclosed in the European patent application 99 104 050.2. The pBLCAT3-vector bearing the -643/+33 GPIIb promoter was obtained from G. Uzan (Uzan et al., 1991). This promoter was sorted from the pBLCAT3-GPIIb vector after HindIII BamHI digestion (Promega, Charbonniéres, France) and was introduced in pcDNA3.1 vector (Invitrogen, Groningen, The Netherlands) opened by the same enzymes. This construct was then deleted of the CMV promoter by MIuIClaI digestion, and the construct obtained was so called pcDNA3-GPIIb. The pTracer^{™}-EF C vector was obtained from Invitrogen (Groningen, The Netherlands). This vector is bearing a Zeozine™ resistance gene.

*Cell culture:* HEL92.1.7 was obtained from ECACC (Sophia Antipolis, France). The cells were maintained in RPMI/10% FCS medium with 5% CO₂. For stable transfections with pcDNA3 constructs, HEL cells (1 x 10⁶ cells) were transfected with 2 µg of Pvul linearized plasmid using 6 µl FUGENE™ 6 (Roche Diagnostics, Meylan, France) during 5 hours. After incubation, the cells were harvested and placed in fresh medium supplemented with 0,6 mg/ml geneticin (Gibco BRL, Cergy Pontoise, France).

Dami cells were maintained in RPMI/10% FCS medium with 5% CO₂. For stable transfections with pTracer constructs, Dami cell (1 x 10⁶ cells) were transfected with 2 µg of Pvul linearized plasmid using 6 µl FUGENE™ during 5 hours. The cells were then harvested and placed in fresh medium. Zeocin™ (Invitrogen, Groningen, The Netherlands) was subsequently added at a final concentration of 300 µg/ml.

*Cell Inductions:* To compare FVIII production, the resistant cells (2,5 x 10⁵ cells/ml) were placed in RPMI/1% BSA with human vWF ± PMA 1 nM. After 4 days of incubation, the cells were numbered and the supernatants were harvested. The supernatants were concentrated on Microsep™ microconcentrators (Pall Gelman Sciences, France) with a 30Kd cut-off. The cells were lysated in Hepes 20 mM, KCl 0,1 M, MgCl₂ 2 mM, Triton X 100 0,5%. Protein concentrations were measured using Bio-Rad D_{c} Protein Assay (Bio-Rad, Ivry sur Seine, France). FVIII productions were measured using FVIII ELISA kit (Asserachrom FVIII, Stago Asnières, France). Concentrated culture media were tested for coagulation activity using a chromogenic FVIII assay (Coamatic FVIII, Biogenic, France).
*RT-PCR and PCR:* Reverse transcriptase (RT) reactions were realized with 2 µg mRNA (extracted with Rneasy Mini kit; QIAGEN S.A., France) using the Super-script™II Rnase H Reverse transcriptase (Gibco BRL, Cergy Pontoise, France) and oligo(dT)¹⁵ primer (Promega, Charbonnieres, France). For PCR, Expand™ long template PCR system (Roche Diagnostics, Meylan, France) was used with 4 µl of each RT product or 10 ng of each control plasmid. Intron splicing was studied using a set of primers specific for intron 1 location and another set for intron 13 location. The first PCR gives a 1701 bp fragment without the intronic sequence and a 2014 bp fragment with the FIX intron 1 sequence. With the 2 other primers, the size of PCR fragments was 623 bp and 935 bp depending upon the absence or the presence of intron 13, respectively. RT-PCR and PCR fragments were run on 0,8% agarose gel and were compared with the 1 Kb ladder (Gibco BRL, Cergy Pontoise, France).

*Immunoprecipitations and FVIII immunoblot analysis:* Before immunoprecipitations, lysates were incubated with human vWF (2,000 ng vWF for 40 ng FVIII; Diagnostica Stago, Asnières, France) for 10 min at room temperature. Fifty microliters of anti human vWF antibody beads provided by Aventis Behring (USA) were added to the samples and incubated overnight at 4°C. The beads were then collected after centrifugation (2 min at 2500 rpm), washed three times with the equilibration buffer (Hepes 10 mM, KCI 100 mM, MgCl₂ 2 mM, Trition x100 0,1%) and diluted in Laemmli buffer (Laemmli, 1970). Samples were then subjected to electrophoresis on SDS-PAGE/7% polyacrylamide gel and semi-dry blotted onto Hybond™ C Pure membrane (Amersham Pharmacia Biotech Europe GmbH, France) The immunoblots were blocked with TBS-T (Tris-HCI 10 mM pH 7,5, NaCl 0,15 M, Tween 0,1%) for 1 h at room temperature and then incubated with 1:3,000 dilution of a sheep anti-human FVIII antibody (Cedarlane, Ontario, Canada). The membrane was then washed 3 times in TBS-T and incubated with a 1:10.000 dilution of a peroxydase-labeled anti-sheep antibody (Dako S.A., Trappes, France) for 30 min. After 3 washes, chemiluminescent signal was detected by autoradiography using the ECL System (Amersham Pharmacia Biotech Europe GmbH, France).

The following results were obtained:

### 1. FVIII expression under CMV promoter control

### 1.1 FVIII production in HEL cells

In order to compare FVIII productions with the same CMV promoter, HEL cells were stably transfected with pcDNA3-FVIII and pcDNA3-FVIII I1+13. pcDNA3.1 vector was used as negative control (Invitrogen, Groningen, The Netherlands). G418-resistant cells were thereafter compared.

No FVIII was detected in the supernatants of lysates of the pcDNA3.1 control cells. Without PMA stimulation, FVIII was detectable neither in the supernatants nor in the lysates of HEL cells. When 1 nM PMA was added to the cell culture, FVIII was detected in the CMV-FVIII- and CMV-FVIII I1+13-expressing cells (Table 1). In the supernatants, FVIII production was 13-fold higher with the CMV-FVIII I1+13 transfected cells compared to the CMV-FVIII-expressing cells. The intracellular FVIII amount was also higher in the CMV-FVIII I1+13 cells (2,5-fold increase).

**Table 1: FVIII production by PMA-stimulated HEL cells. 5 x 10⁵ G418-resistant HEL cells were placed in 2 ml RPMI/1% BSA/1 nM PMA for 4 days. The supernatants were thereafter harvested and the cells were suspended in 250 µl of lysis buffer. FVIII was quantified using FVIII ELISA kit. Results are expressed as the mean values ± SEM of 4 individual experiments. pcDNA3 was used as reference for statistics (** represents p<0,01).**

| | **pcDNA3** | **CMV-FVIII** | **CMV-FVIII I1+13** |
|---|---|---|---|
| Supernatants (ng/ml) | 0 ± 0 | 0,22 ± 0,05 | 2,97 ± 0,26 ** |
| Lysates (ng/mg of proteins) | 0 ± 0 | 1,38 ± 0,22 | 3,77 ± 0,26 ** |

### 1.2 FVIII coagulant activity

CMV-FVIII 1+13 HEL cells were placed in induction conditions with 1 nM PMA. After 4 days, the conditioned media were harvested, concentrated and FVIII antigen and FVIII coagulant activity were then quantified. The results demonstrated that HEL-produced FVIII was an active molecule (Table 2). The mean specific activity was 4.858,9 ± 798,8 U/mg that was very similar to the specific activity of plasma FVIII.

**Table 2: FVIII coagulant activity in the supernatants of PMA stimulated CMV-FVIII I1+13 HEL cells. CMV-FVIII I1+13 HEL cells were placed in the induction conditions for 4 days. FVIII antigen and FVIII coagulant activity were then quantified in the concentrated supernatants. Two independent experiments were presented. The results are expressed as the mean values ± SEM (n = 3).**

| | **FVIII Antigen** **(ng/ml)** | **Coagulation activity** **(mU/mL)** | **Specific activity** **(U/mg)** |
|---|---|---|---|
| Exp. 1 | 6,24 ± 0,70 | 33,58 ± 8,15 | 5.335,0 ± 882,7 |
| Exp. 2 | 12,04 ± 0,62 | 52,85 ± 6,64 | 4.382,9 ± 369,2 4.858,9 ± 798,8 |

### 1.3 Splicing of FIX intron 1

Transfected HEL cells were incubated in RPMI-1% BSA-1 nM PMA medium during 3 days. RNAs were extracted and used for RT-PCR reactions. In pcDNA3-transfected HEL cells, no fragment was obtained with the 2 sets of primers. The 1701 and 623 bp fragments were essentially detected in the RT-PCR realized with mRNA from CMV-FVIII I1+13 transfected HEL cells demonstrating that the intronic sequences were correctly spliced. Conversely, the control plasmid pcDNA3-FVIII I1+13 exhibited the 2014 bp and 935 bp fragments, corresponding to the detection of introns 1 and 13.

### 1.4 FVIII Immunoblot analysis

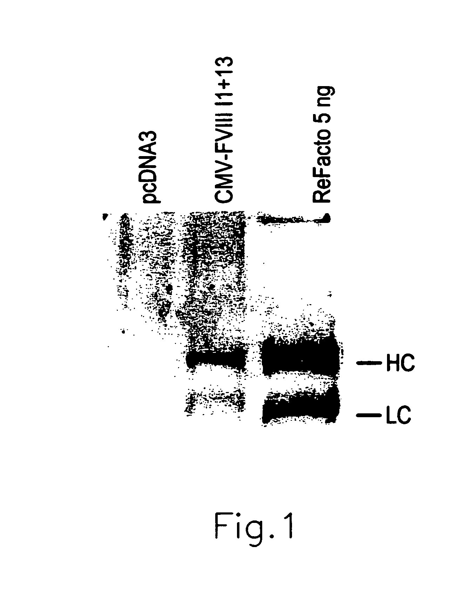

To further analyse FVIII recombinant proteins in HEL supernatants. FVIII was purified using beads coupled with an anti-human vWF antibody (provided by Aventis Behring). PcDNA3 and pcDNA3-FVIII I1+13 transfected HEL cells were incubated in RPMI-1% BSA-1 nM PMA medium supplemented with hu vWF (150 ng/mL). Supernatants were then concentrated on Microsep™ microconcentrators with a 30 Kd cut-off. The immunoprecipited proteins were then subjected to electrophoresis and FVIII immunoblot analysis. ReFacto®, a therapeutic recombinant B-domain deleted FVIII (Wyeth Genetics Institute) was used as control FVIII. The result is presented in Fig. 1. The immunoblot analysis detected both the FVIII light and heavy chains and demonstrated that the recombinant FVIII produced by CMV-FVIII I1+13 HEL cells presented the same protein profile as ReFacto®.

### 2. FVIII expression under GPIIb promoter control

### 2.1 Obtention of pTracer-GPIIb constructs

The lineage-specific promoter GPIIb was chosen to express FVIII transgenes in the hematopoietic cell line Dami. The pBLCAT vector bearing the -643/+33 GPIIb promoter was obtained from G. Uzan (Uzan, 1991 #44). This promoter was sorted from the pBLCAT-GPIIb vector after HindIII-BamHI digestion (Promega, Charbonnières, France). It was introduced in pcDNA3.1 vector (Invitrogen, Leek, The Netherlands) opened by the same enzymes. This construct was then deleted of the CMV promoter by Mlul-Clal digestion, and was so called pcDNA3-GPIIb. This GPIIb vector bearing the -597/+33 GPIIb promoter is already disclosed in the European patent application 99 107 397.4.

PTracer™-EF C was opened by Nrul-Spel digestion. This enzyme digestion deleted the EF-1α promoter from the initial vector. The same digestion was used to extract the GPIIb promoter from pcDNA3-GPIIb plasmid. The 2 fragments were then ligated and the resulting expression plasmid was called pTracer/GPIIb.

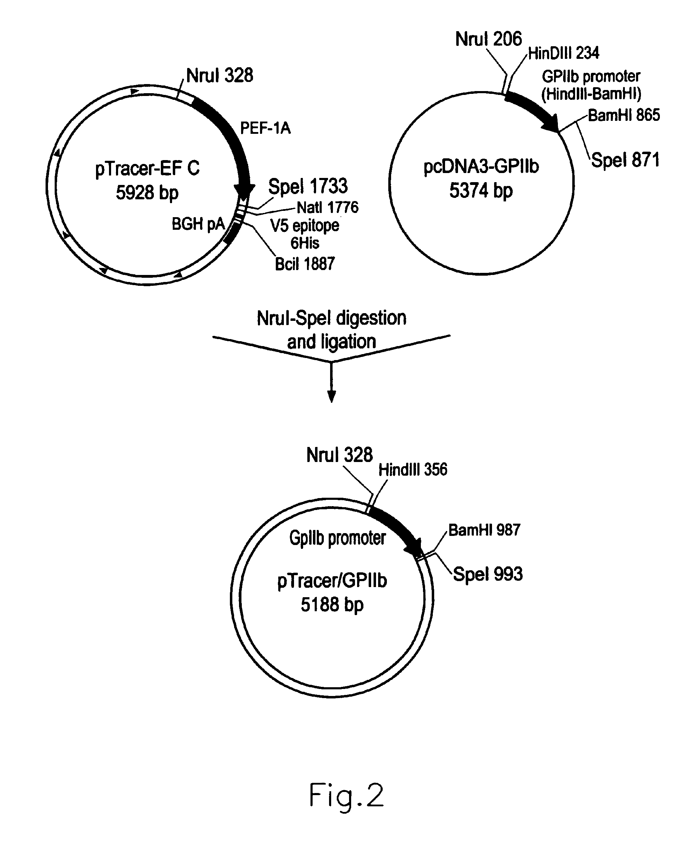

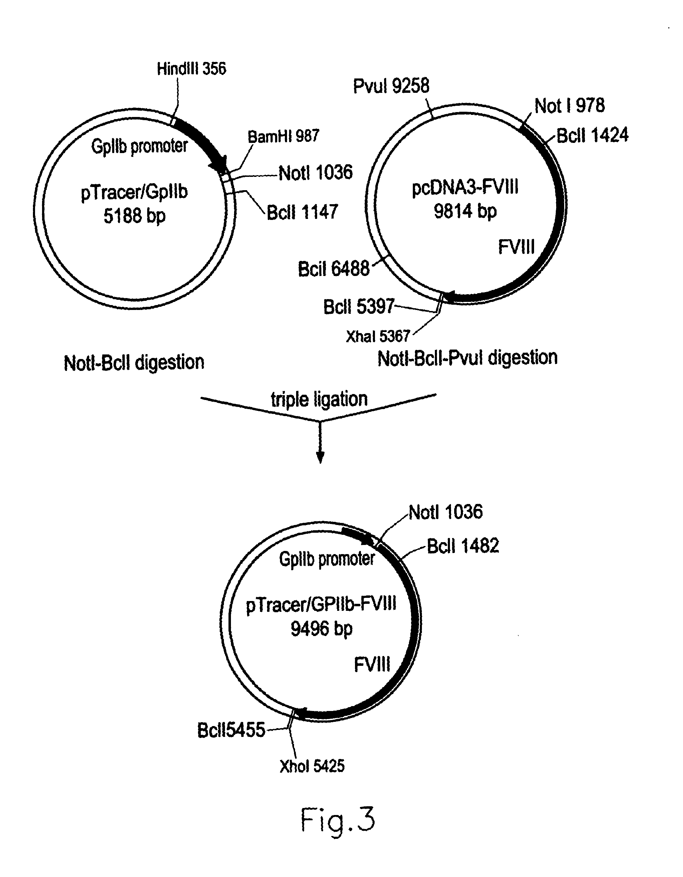

In order to obtain pTracer/GPIIb-FVIII, pTracer/GPIIb was digested by NotI and BcII (Fig. 3). This digestion eliminated V5 epitope and the polyhistidine region of the initial pTracer-EF C vector (Fig. 2). pcDNA3-FVIII was treated by NotI, BcII and Pvul. The 2 resulting FVIII fragments (446 bp and 3973 bp) were extracted from agarose gel, and pTracer/GPIIb-FVIII was obtained with a triple ligation. The same strategy was used for pTracer/GPIIb-FVIII I1+13.

### 2.2 FVIII production in Dami cells

Dami cells were stably transfected using Zeocin selection. PTracer/GPIIb was used as negative vector control. Three pools were obtained for each GPIIb constructs. The cells were placed in induction conditions and FVIII was quantified in both the supernatants and the cell lysates.

In all the lysates no FVIII was produced in Dami cells transfected with pTracer/GPIIb. Conversely, FVIII was detected in the lysates of GPIIb-FVIII and GPIIb-FVIII I1+13 Dami cells (Table 3). Without PMA stimulation, GPIIb-FVIII I1+13-expressing cells produced about 25-fold higher FVIII than GPIIb-FVIII-expressing Dami cells. When the cells were incubated with PMA, the FVIII production was increased (6-fold increase for GPllb-FVIII-transfected cells and 4-fold increase with the GPIIb-FVIII I1+13-expressing cells). The difference in FVIII production between FVIII and FVIII I1+13-transfected Dami cells was statistically significant (p<0,05 without PMA and p<0,01 with PMA).

In the supernatants, no FVIII was detected with Dami cells transfected with pTracer/GPIIb. Without PMA, the GPIIb-FVIII I1+13-expressing cells produced about 7,5-fold higher FVIII than the GPIIb-FVIII-expressing cells. A significant increase in FVIII production was measured in the supernatants following PMA stimulation. However, GPIIb-FVIII I1+13-expressing cells produced always more FVIII than the GPIIb-FVIII Dami cells.

These results demonstrated that Dami cells transfected with GPIIb-FVIII I1+13 produced significantly more FVIII than the GPllb-FVIII-expressing cells.

**Table 3: FVIII production (ng FVIII/mg of proteins) in Dami cell lysates.5 x 10⁵ Zeocin-resistant Dami cells were placed in 2 ml RPMI/1% BSA ± 1 nM PMA for 4 days. The supernatants were thereafter harvested and the cells were suspended in 250 µl of lysis buffer. FVIII was quantified using FVIII ELISA kit. Results are expressed as the mean values ± SEM of 3 individual experiments. pTracer/GPIIb was used as reference for statistics (** represents p<0,01).**

| | **PTracer/GPIIb** | **GPIIb-FVIII** | **GPIIb-FVIII I1+13** |
|---|---|---|---|
| Without PMA | 0 ± 0 | 0,62 ± 0,37 | 15,81 ± 11,06 ** |
| With 1 nM PMA | 0 ± 0 | 3,66 ± 2,19 | 62,92 ± 28,27 ** |

### 2.3 FVIII coagulant activity

The FVIII coagulant activity was measured in concentrated supernatants using a chromogenic test. The results are presented in Table 4. No coagulant activity was detected in the supernatants from Dami cells transfected with GPIIb (data not shown). Conversely, a FVIII coagulant activity was found in the supernatants of GPIIb-FVIII I1+13-expressing Dami cells, and the specific activity was calculated to be 4.622,3 ± 1.061,4 U/mg. The correlation between FVIII antigen and FVIII coagulant acitivity demonstrated that recombinant FVIII produced in Dami cells was a biologically active FVIII.

**Table 4: FVIII coagulant activity in supernatants of PMA-stimulated GPIIb-FVIII I1+13 Dami cells.GPIIb-FVIII I1+13 Dami cells were placed in the induction conditions for 4 days. FVIII antigen and FVIII coagulant acitivity were then quantified in the concentrated supernatants. The results are expressed as the mean values ± SEM (n = 5).**

| | **FVIII Antigen** **(ng/ml)** | **Coagulation activity** **(mU/ml)** | **Specific activity** **(U/mg)** |
|---|---|---|---|
| Concentrated supernatants | 29,96 ± 5,28 | 142,53 ± 53,12 | 4.622,3 ± 1.061,4 |

### 2.4 FIX intron 1 splicing

In order to verify the correct splicing of the 2 FIX intron 1 sequences, RT-PCR analysis was realized. mRNAs were extracted from the unstimulated or PMA-stimulated transfected cells after 3 days of induction (Rneasy Mini Kit; Qiagen, Courtaboeuf, France). A PCR using pTracer/GPIIb-FVIII and pTracer/GPIIb-FVIII I1+13 were used as controls. RT-PCR results demonstrated that the FVIII mRNA was essentially spliced in GPIIb-FVIII I1+13 Dami cells. Dami cells were therefore able to correctly process FVIII mRNAs.

### 2.5 FVIII immunoblot analysis

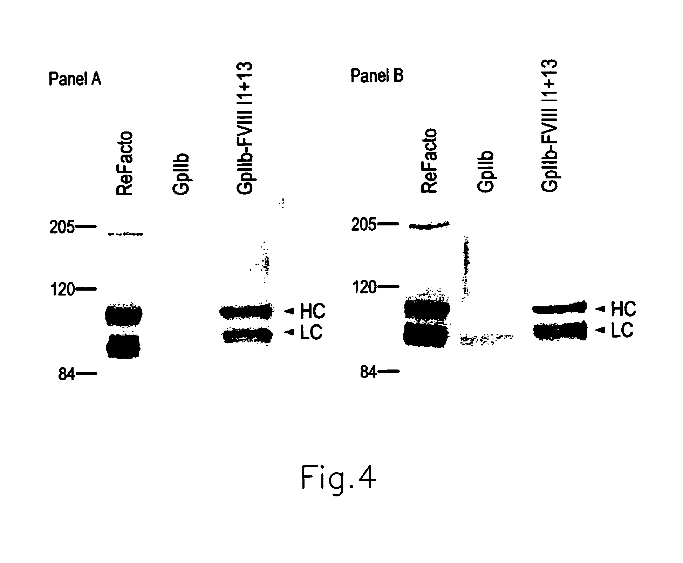

To further analyse FVIII protein profile, beads coupled with an anti-human vWF monoclonal antibody were used (provided by Aventis Behring). Cell inductions were realized with human vWF (400 ng/ml) in the induction medium. The supernatants were concentrated and immunoprecipitated with the beads bearing an anti-human vWF monoclonal antibody. For the cell lysates, human vWF was added to the samples just before immunoprecipitations with the anti-human vWF antibody (Without adding human vWF after cell lysis, we were unable to immunoprecipitate FVIII). In pTracer/GPIIb Dami cells lysates or supernatants, no FVIII was detected (Fig. 4).

The recombinant FVIII produced by the GPIIb-FVIII I1+13-transfected Dami cells presented a protein profile very similar to the therapeutic recombinant B-domain-deleted FVIII (ReFacto®, Wyeth Genetics Institute).

The results of the present invention were obtained with 2 cell lines, HEL and Dami. These hematopoietic cell lines are able to produce a biologically active recombinant FVIII. This in vitro-produced FVIII presents a correct protein profile, essentially similar to a therapeutic B domain-deleted recombinant FVIII (ReFacto®, Wyeth Genetics Institute). The presence of 2 factor IX truncated introns in the FVIII I1+13 construct is responsible for a dramatic increase in FVIII production, and confirmed an ubiquitous effect of these FIX intron 1 sequences. The results obtained with GPIIb constructs-transfected Dami cells demonstrated that GPIIb promoter is able to efficiently improve the tissue-specific production of recombinant B domain-deleted FVIII in Dami cells. These results confirm that the GPIIb promoter controls the specific production of coagulation factor in hematopoietic cells and specifically in megakaryocytic oriented cells.

### Legend to Fig. 1 to Fig. 4

**Fig. 1****: Immunoblot analysis of the immunoprecipited FVIII produced by the transfected HEL cells.** PcDNA3- and CMV-FVIII I1+13 HEL cells were incubated with human vWF and PMA for 4 days. Supernatants were concentrated and thereafter subjected to vWF immunoprecipitation. FVIII immunoblot analysis was realized using an anti-human FVIII antibody and detected the light chain (LC) and the heavy chain (HC).

**Fig. 2****: Obtention of the pTracer/GPIIb vector.** pTracer-EF C and pcDNA3-GPIIb vectors were digested by Nrul and SpeI. The opened pTracer and the Nrul-Spel GPIIb promoter were then ligated in order to obtain pTracer/GPIIb plasmid. Legendes: PEF-1α human elongation factor 1α promoter: 6His, Polyhistidine region; BGH pA, Polyadenylation region.
**Fig. 3****: Obention of the pTracer/GPIIb-FVIII vector.** pTracer/GPIIb vector was digested by NotI and BcII. FVIII cDNA was cloned between NotI and Xhol. PcDNA3-FVIII was digested by NotI, BcII and Pvul. The opened pTracer/GPIIb and the 2 FVIII fragments were ligated in order to obtain pTracer/GPIIb-FVIII plasmid.

**Fig. 4****: Immunoblot analysis of the immunoprecipited FVIII produced by the transfected Dami cells.** PTracer/GPIIb- and GPIIb-FVIII I1+13 Dami cells were incubated with human vWF and PMA for 4 days. Supernantants were concentrated and thereafter subjected to vWF immunoprecipitation. Lysates were incubated with human vWF before immunoprecipitations. FVIII immunoblot analysis was realized using an anti-human FVIII antibody and detected the light chain (LC) and the heavy chain (HC). ReFacto® (5 ng) was used as control.

### References:

1. Andersson, L.O., Forsman, N., Huang, K., Larsen, K., Lundin, A., Pavlu, B., Sandberg, H., Sewerin, K., Smart, J. - Isolation and characterization of human factor VIII: molecular forms in commercial factor VIII concentrate, cryoprecipitate, and plasma. Proc Natl Acad Sci U S A 1986 May; 83(9):2979-83.
2. Ballen, K.K., Pitchie, A.J., Murphy, C., Handin, R.I., Ewenstein, B.M. - Expression and activation of protein kinase C isoforms in a human megakaryocytic cell line. Exp Hematol 1996 Nov.; 24(13:1501-8.
3. Gitschier, J., Wood, W.I., Garalka, T.M., Wion, K.L., Chen, E.Y., Eation D.H., Vehar, G.A., Capan, D.J., Lawn, R.M. - Characterization of the human factor VIII gene. Nature 1984 Nov. 22-28; 312(5992):326.30.
4. Greenberg, S.M., Rosenthal, D.S., Greeley, T.A., Tantravahi, R., Handin, R.I.-Characterization of a new megakaryocytic cell line: the Dami cell. Blood 1988 Dec.; 72(6):1968-77.
5. Hayward, C.P., Bainton, D.F., Smith, J.W., Horsewood, P., Stead, R.H., Podor, T.J., Warkentin, T.E., Kelton, J.G. - Multimerin is found in the alpha-granules of resting platelets and is synthesized by a megakaryocytic cell line. J Clin Invest 1993 Jun.; 91(6):2630-9.
6. Hill, S.A., Shaughnessy, S.G., Joshua, P., Ribau, J., Austin, R.C., Podor, T.J. - Differential mechanisms targeting type 1 plasminogen activator inhibitor and vitronectin into the storage granules of a human megakarykocytic cell line. Blood 1996 Jun.; 15:87(12):5061-73.
7. Laemmli, U.K. - Cleavage of structural proteins during the assembly of the head of bacteriophage T4. Nature 1970 Aug. 15:227(259):680-5.
8. Lang, M.W., Heffner, C.H., Williams, J.L., Peters, C., Prochawnik, E.V. - Regulation of megakaryocyte phenotype in human erythroleukemia cells. J Clin Invest 1990 Apr.; 85(4) :1072-84.
9. Martin, P., Papayannopoulou, T. - HEL cells: a new human erythroleukemia cell line with spontaneous and induced globin expression. Science 1982 Jun. 11; 216(4551):1233-5.
10.Tabilio. A., Rosa, J.P., Test, U., Kieffer, N., Nurden, A.T., Del Canizo, M.C. Bretan-Garius, J., Vainchenker, W. - Expression of platelet membrane glycoproteins and alpha-granule proteins by a human erytholeukemia cell line (HEL). EMBO J 1984 Feb; 3(2):453-9.
11. Toole, J.J., Knopf, J.L., Wozney, J.M., Sultzman, L.A., Buecker, J.L., Pittman, D.D., Kaufman, R.J., Brown, E., Shoenaker, C., Orr, E.C. et al. - Molecular cloning of a cDNA encoding human antihaemophilic factor. Nature 1984, Nov. 22-28; 312(5992):342-7.
12. Uzan, G., Prenant, M., Prandini, M.H., Martin, F., Marguerie, G. - Tissue-specific expression of the platelet GPIIb gene. J Biol. Chem 1991 May 15; 226(14):8932-9.
13.velar, G.A., Keyt, B., Eaton, D., Rodriguez, H., O'Brien, D.P., Rotblat, F., Oppermann, H., Keck, R., Wood, W.I., Harkins, R.N. et al. - Structure of human factor VIII. Nature 1984 Nov. 22-28; 312(5992):337-42.
14.von der Vuurst, H., Hendriks, M., Lapetina, E.G., van Willigen, G., Akkerman, J.W. - Maturation of megakaryoblastic cells is accompanied by upregulation of G(s)alpha-L subtype and increased cAMP accumulation. Thromb Haemost 1998 May; 79(5):1014-20.

## Claims

1. Factor VIII cDNA wherein the B-domain of the wild-type factor VIII cDNA has been deleted and wherein a truncated factor IX intron I has been inserted into the factor VIII introns 1 and 13, **characterized in that** it contains, as a promoter, the tissue-specific promoter of the glycoprotein IIb (GPIIb).

2. Factor VIII cDNA as claimed in Claim 1, **characterized in that** said tissue-specific promoter of the glycoprotein IIb is the promoter of the human platelet glycoprotein IIb (GPIIb).

3. Process for the production of Factor VIII, **characterized in that** the production is performed in the cell line HEL or in the cell line Dami using the Factor VIII cDNA as claimed in claim 1.

4. Process as claimed in claim 3, **characterized in that** the production of Factor VIII is stimulated by an inducer,

5. Process as claimed in claim 4, **characterized in that** as inducer phorbol-12-myristate-13-acetate (PMA) is used.

## Patentansprüche

1. Faktor VIII cDNA worin das B-Gebiet des wild-typs Faktor VIII cDNA entfernt wurde und worin in die Faktor VIII introns 1 und 13 ein abgestumpftes Faktor IX intron 1 eingesetzt wurde, **dadurch gekennzeichnet dass** dieses, als Promotor, den Gewebe-spezifischen Promotor der Glycoproteine IIb (GPIIb) enthält.

2. Faktor VIII cDNA wie im Anspruch 1 beansprucht, **dadurch gekennzeichnet, dass** der obengenante gewebe-spezifische Promotor der Glycoproteine IIb der Promotor der Glycoproteine IIb (GPIIb) des menschlichen Blutplättchens ist.

3. Verfahren zur Produktion von Faktor VIII, **dadurch gekennzeichnet, dass** die Produktion in der Zelllinie HEL oder mit Benutzung der Faktor VIII cDNA in der Zelllinie DAMI, wie im Anspruch 1 beansprucht, hergestellt wird.

4. Verfahren wie im Anspruch 3 beansprucht, **dadurch gekennzeichnet, dass** die FaktorVIII Produktion durch einen Induktor stimuliert ist.

5. Verfahren wie im Anspruch 4 beansprucht, **dadurch gekennzeichnet, dass** als Induktor phorbol-12 myristate-13-azetat (PMA) benutzt wird.

## Revendications

1. ADNc de facteur VIII dont a été enlevé le domaine B de la ADNc de facteur VIII de type sauvage et dont, dans les introns 1 et 13 du facteur VIII, a été inséré un intron 1 du facteur IX tronqué, **caractérisé en ce que** y est contenu, en tant que promoteur, le promoteur spécifique de tissu de la glycoprotéine IIb (GPIIb).

2. ADNc de facteur VIII ainsi que revendiqué dans la revendication 1, **caractérisé en ce que** ledit promoteur spécifique de tissu de la glycoprotéine IIb est le promoteur de la glycoprotéine IIb des plaquettes humaines (GPIIb).

3. Procédé de production de facteur VIII, **caractérisé en ce que** la production est effectuée dans la lignée cellulaire HEL ou dans la lignée cellulaire Dami grâce à l'emploi de ADNc de facteur VIII ainsi que revendiqué dans la revendication 1.

4. Procédé tel que revendiqué dans la revendication 3, **caractérisé en ce que** la production de facteur VIII est stimulée par un inducteur.

5. Procédé tel que revendiqué dans la revendication, **caractérisé en ce que** on utilise en tant qu'inducteur du phorbol-12 myristate-13-acetate (PMA).
